Europäisches Patentamt

European Patent Office

Office européen des brevets

(11) Numéro de publication: **0 552 076 B1**

(12) **FASCICULE DE BREVET EUROPEEN**

(45) Date de publication du fascicule du brevet: **11.10.95** (51) Int. Cl.⁶: **C07C 17/26**, C07C 19/08

(21) Numéro de dépôt: **93400037.3**

(22) Date de dépôt: **08.01.93**

(54) **Synthèse d'iodures de perfluoroalkyle.**

(30) Priorité: **13.01.92 FR 9200261**

(43) Date de publication de la demande:
**21.07.93 Bulletin 93/29**

(45) Mention de la délivrance du brevet:
**11.10.95 Bulletin 95/41**

(84) Etats contractants désignés:
**BE DE ES FR GB IT NL**

(56) Documents cités:
**FR-A- 1 415 498**
**US-A- 3 404 189**

(73) Titulaire: **ELF ATOCHEM S.A.**
**4 & 8, Cours Michelet**
**La Défense 10**
**F-92800 Puteaux (FR)**

(72) Inventeur: **Bertocchio, René**
**49 Bis Rue des Vallières**
**F-69390 Vourles (FR)**
Inventeur: **Lacote, Georges**
**Lieu Dit Revol**
**F-71170 Chauffailles (FR)**
Inventeur: **Verge, Christophe**
**38 Allée Fernand Léger**
**F-27300 Bernay (FR)**

(74) Mandataire: **Leboulenger, Jean et al**
**Elf Atochem S.A.**
**Département Propriété Industrielle, Cedex 42**
**- La Défense 10**
**F-92091 Paris la Défense (FR)**

**Description**

La présente invention concerne le domaine des hydrocarbures aliphatiques perhalogénés et a plus particulièrement pour objet la préparation des iodures de perfluoroalkyle RfI dont le radical perfluoroalkyle Rf, linéaire ou ramifié, contient de 4 à 12 atomes de carbone.

Les iodures de perfluroalkyle correspondant à la formule brute $C_nF_{2n+1}I$ où n désigne le nombre d'atomes de carbone (ou degré de condensation en carbone) sont utilisés comme intermédiaires de synthèse pour de nombreuses applications concernant d'une manière générale le domaine des substances tensio-actives fluorées et plus particulièrement les bases pour formulations extinctrices, les apprêts hydrophobes et oléophobes pour le traitement des textiles ou du papier, et plus récemment pour des applications à caractère médical (agents de contraste ou transporteurs d'oxygène).

Les iodures de perfluoroalkyle sont obtenus par télomérisation du tétrafluoroéthylène (taxogène) par l'iodure de pentafluoroéthyle $C_2F_5I$ (télogène), lui-même préparé par action de l'iode et du pentafluorure d'iode sur le tétrafluoroéthylène en présence d'un catalyseur. Ces deux réactions peuvent être couplées comme décrit dans le brevet FR 1 385 682, mais dans la plupart des cas on prépare d'abord $C_2F_5I$ et on l'utilise ensuite dans la télomérisation qui conduit aux iodures de perfluoroalkyle linéaires. Pour accéder aux iodures de perfluoroalkyle ramifiés, on utilise comme télogène un iodure de perfluoroalkyle secondaire tel que l'iodure d'heptafluoroisopropyle $CF_3CFICF_3$.

La réaction de télomérisation peut être réalisée selon au moins trois méthodes dont la différence essentielle réside dans le mode d'activation qui peut être :
- soit radicalaire à l'aide de divers amorceurs peroxydiques comme dans les procédés faisant l'objet des brevets FR 2 035 913, FR 2 325 665 et US 3 226 449,
- soit catalytique par l'intervention d'un système rédox comme dans les procédés selon les brevets FR 2 028 781 et FR 2 098 335,
- soit enfin thermique comme dans les procédés faisant l'objet des brevets FR 1 415 498 et US 3 404 189.

Dans tous ces procédés on obtient une distribution plus ou moins large des différentes longueurs de chaîne et, même dans les procédés à initiation catalytique réputés être plus sélectifs, il est difficile de parvenir à des distributions relativement étroites pour des télomères de rang i allant de 2 à 5, i désignant le nombre de molécules de tétrafluoroéthylène télomérisées par le télogène.

Quand ces iodures de perfluoroalkyle sont utilisés comme intermédiaires de synthèse, les télomères les plus lourds (ceux de rang i > 5) sont moins solubles dans les milieux réactionnels classiques et présentent des cinétiques de réaction nettement plus lentes que les télomères les plus légers ($1 \leq i \leq 5$). Il en résulte une accumulation défavorable d'intermédiaires non transformés tout au long de la chaîne de réactions conduisant au produit final, avec une incidence directe sur la qualité de ce dernier notamment dans les applications faisant appel à ses propriétés physico-chimiques comme par exemple la tensio-activité.

Le brevet GB 1 314 668 décrit un procédé de séparation des télomères ayant un degré de condensation en carbone inférieur à 14 par extraction à l'aide d'un solvant. Cette méthode entraîne une perte de produits et n'est donc pas satisfaisante sur le plan économique.

Un procédé pour réduire le taux de télomères lourds (>C12) consiste à augmenter le rapport télogène/taxogène (voir les brevets FR 2 028 781 et FR 2 035 913), mais cela implique un faible taux de conversion du télogène avec des taux de recyclage importants et conduit à une distribution orientée sélectivement vers le télomère de rang 1. Ainsi, selon le brevet FR 2 035 913, la télomérisation de $C_2F_4$ par $C_2F_5I$ dans un rapport $C_2F_5I/C_2F_4$ égal à 2,9 conduit à la distribution suivante :

| <u>**RANG**</u> | <u>**TELOMERE**</u> | <u>**%**</u> |
|:---:|:---:|:---:|
| 1 | $C_4F_9I$ | 42,2 |
| 2 | $C_6F_{13}I$ | 23,8 |

| 3 | $C_8F_{17}I$ | 14,6 |
| 4 | $C_{10}F_{21}I$ | 8,64 |
| 5 | $C_{12}F_{25}I$ | 6,25 |
| $\geq$ 6 | $\geq C_{14}$ | 4,52 |

On peut également agir sur la durée de réaction et le taux de conversion du taxogène. D'une manière générale, les faibles taux de conversion améliorent la sélectivité en télomère de rang 1. En pratique, la multiplicité des étapes qui seraient nécessaires pour parvenir au télomère de rang i de manière sélective conduit à une technologie complexe et à un résultat équivalent en ce qui concerne le taux de produits lourds. Dans les procédés opérant en continu, l'optimisation sur un télomère de rang i est réalisée en recyclant totalement ou partiellement les télomères de rang i-k avec $1 \leq k \leq i-1$.

Or, de nombreuses applications font appel à des télomères appartenant à la coupe $C_6-C_{12}$ et plus particulièrement $C_8-C_{10}$. Les procédés ci-dessus ne permettent pas d'optimiser la production de télomères RfI sur cette coupe sans accroître inévitablement la concentration en produits lourds.

L'objectif de la présente invention est donc de diminuer la proportion de télomères non valorisables (télomères en $C_{14}$ et au delà).

Il est par ailleurs bien connu que la télomérisation des iodures de perfluoroalkyle est une télomérisation bien particulière dans le sens où chacun des télomères formés peut à son tour agir comme télogène et contribuer ainsi à l'allongement des chaînes, fonction qui en télomérisation classique est à peu près exclusivement assurée par les réactions de propagation.

En réalisant des télomérisations du tétrafluoroéthylène à partir d'iodures de perfluoroalkyle à différents degrés de condensation en carbone, il a été constaté avec surprise que l'iodure $C_2F_5I$ conduit à des distributions beaucoup plus étalées que celles obtenues avec ses homologues supérieurs tels que $C_4F_9I$, $C_6F_{13}I$, etc... Ainsi, la télomérisation du tétrafluoroéthylène effectuée en prenant successivement $C_2F_5I$, $C_4F_9I$ et $C_6F_{13}I$ comme télogène conduit, pour un rapport molaire télogène/$C_2F_4$ de 2,5 et toutes choses égales par ailleurs, aux distributions suivantes :

| Télogène de départ | Télomères formés (% molaire) de rang : | | | | |
|---|---|---|---|---|---|
| | 1 | 2 | 3 | 4 | 5 |
| $C_2F_5I$ | 67,5 | 21,9 | 7,1 | 2,3 | 0,8 |
| $C_4F_9I$ | 75,3 | 18,8 | 4,5 | 1,1 | 0,3 |
| $C_6F_{13}I$ | 77,4 | 17,8 | 3,8 | 1,0 | <0,1 |

Partant de cette observation, il a maintenant été trouvé que, dans la préparation continue des iodures de perfluoroalkyle par télomérisation thermique du tétrafluoroéthylène par l'iodure de pentafluoroéthyle ou d'heptafluoroisopropyle, il est possible de réduire la proportion de télomères lourds indésirables par une alimentation étagée du tétrafluoroéthylène, c'est-à-dire en introduisant en tête du réacteur une fraction seulement du tétrafluoroéthylène, le complément étant introduit en un point du réacteur où la concentration du mélange réactionnel en homologues supérieurs est devenue suffisante pour accélérer les réactions de transfert.

Le procédé selon l'invention, mis en oeuvre dans un réacteur tubulaire, est donc caractérisé en ce qu'il comprend une double alimentation en tétrafluoroéthylène, la première en tête du réacteur et la seconde en au moins un point du réacteur situé entre les 2/5 et les 3/4 de la longueur du tube, de préférence situé environ aux 2/3 du tube.

Bien qu'on préfère effectuer la seconde alimentation de $C_2F_4$ en un seul point situé entre les 2/5 et les 3/4 du tube, on ne sortirait pas du cadre de la présente invention en effectuant cette seconde alimentation de $C_2F_4$ en plusieurs points situés dans ladite zone du tube.

La proportion de tétrafluoroéthylène alimentée en tête du réacteur peut aller de 30 à 75 % (de préférence 40 à 60 %) par rapport à la quantité totale de tétrafluoroéthylène mis en oeuvre.

Le rapport molaire global :

$$\frac{\text{télogène}}{C_2F_4 \text{ total}}$$

peut aller de 1 à 3 et est de préférence compris entre 1,1 et 2.

La réaction de télomérisation peut être conduite dans un domaine de température allant de 300 à 360°C, mais elle est avantageusement effectuée à une température comprise entre environ 325 et 355°C.

Industriellement, on préfère travailler à la pression atmosphérique mais on ne sortirait pas du cadre de la présente invention en travaillant à une pression supérieure à la pression atmosphérique, pourvu que le système réactionnel reste à l'état gazeux.

Un temps de contact compris entre 10 et 70 secondes convient généralement bien, mais industriellement on préfère des temps de contact allant de 30 à 60 secondes.

Dans un procédé continu, les taux de conversion du télogène sont rarement quantitatifs et cela implique un recyclage plus ou moins important du télogène non transformé ou d'un mélange de celui-ci avec des télomères de rang moyen inférieur à celui des télomères désirés. Le procédé selon l'invention s'applique également à ce cas.

Les exemples suivants illustrent l'invention sans la limiter.

## EXEMPLE 1

On utilise un réacteur tubulaire vide en acier inoxydable de 20 m de long et 4 mm de diamètre intérieur. Il est maintenu par un dispositif approprié à une température de 344 ± 5°C et il comporte à mi-longueur un piquage isolable par l'intermédiaire d'une vanne.

**1a** : Ce réacteur est alimenté en tête par un courant gazeux de $C_2F_5I$ (0,349 mole/h) et de $C_2F_4$ (0,277 mole/h). Les produits de la réaction sont recueillis en sortie de réacteur sous forme liquide ou gazeuse par piégeage. Leur analyse montre que 2,3 % des télomères formés (% molaires) présentent une condensation en carbone supérieure à 12.

**1b** : On utilise le même réacteur, mais on introduit en tête la totalité du $C_2F_5I$ et seulement 75 % (0,208 mole/h) du $C_2F_4$. Le complément de $C_2F_4$ (0,069 mole/h) est introduit par le piquage latéral à mi-longueur du tube. Dans ces conditions, la proportion de télomères à condensation en carbone supérieure à 12 tombe à 1,7 %.

**1c** : On répète l'essai 1b en introduisant seulement 50 % du $C_2F_4$ en tête du réacteur avec la totalité du $C_2F_5I$, le reste du $C_2F_4$ entrant par le piquage latéral. Le produit recueilli en sortie du réacteur ne renferme plus que 0,9 % de télomères supérieurs au $C_{12}F_{25}I$.

Le tableau suivant précise les conditions opératoires et les résultats de ces trois essais.

| ESSAI | 1a comparatif | 1b | 1c |
|---|---|---|---|
| % de $C_2F_4$ introduit en tête du réacteur | 100 | 75 | 50 |
| Rapport molaire global $C_2F_5I/C_2F_4$<br>Temps de contact (secondes)<br>Température (°C) | 1,26<br>59<br>344 | 1,22<br>60,5<br>344 | 1,26<br>60,9<br>344 |
| Taux de conversion de $C_2F_4$ (%) | 73,3 | 69,7 | 62,8 |
| Répartition des télomères (% molaires) | | | |
| $C_4$<br>$C_6$<br>$C_8$<br>$C_{10}$<br>$C_{12}$<br>> $C_{12}$ | 53,2<br>24,7<br>11,6<br>5,3<br>2,7<br>2,3 | 54,4<br>24,6<br>11,1<br>6,0<br>2,1<br>1,7 | 56,8<br>24,8<br>10,5<br>5,2<br>1,6<br>0,9 |

## EXEMPLE 2

On répète les trois essais de l'exemple 1, mais en plaçant le piquage latéral aux 2/3 de la longueur du tube (soit à 13,33 m de l'entrée).

En introduisant la moitié du $C_2F_4$ par ce piquage (essai 2c), la teneur en télomères lourds est réduite à 0,7 %.

Le tableau suivant précise les conditions opératoires et les résultats de ces trois essais.

| ESSAI | 2a comparatif | 2b | 2c |
|---|---|---|---|
| % de $C_2F_4$ introduit en tête du réacteur | 100 | 75 | 50 |
| Rapport molaire global $C_2F_5I/C_2F_4$<br>Temps de contact (secondes)<br>Température (°C) | 1,22<br>60,6<br>344 | 1,21<br>60,3<br>344 | 1,25<br>59,5<br>344 |
| Taux de conversion de $C_2F_4$ (%) | 74,25 | 66,3 | 54,2 |
| Répartition des télomères (% molaires) | | | |
| $C_4$<br>$C_6$<br>$C_8$<br>$C_{10}$<br>$C_{12}$<br>> $C_{12}$ | 52,9<br>24,0<br>11,2<br>7,6<br>2,4<br>1,9 | 56,0<br>24,5<br>10,6<br>5,5<br>1,9<br>1,6 | 60,6<br>23,9<br>9,2<br>4,4<br>1,2<br>0,7 |

## EXEMPLE 3

Le réacteur utilisé à l'exemple 2 (piquage latéral aux 2/3) est alimenté par un mélange renfermant 75,8 % de $C_2F_5I$ et 24,2 % de $C_4F_9I$ (% molaires).

**3a** : On opère à 344°C et introduit en tête du réacteur la totalité du tétrafluoroéthylène, le rapport molaire global télogènes/$C_2F_4$ étant sensiblement le même que dans les exemples précédents.

Dans ces conditions, le taux de télomères lourds atteint 1,82 % pour un taux de conversion du $C_2F_4$ égal à 76,6 %.

**3b** : On opère également à 344°C, mais on introduit 50 % du $C_2F_4$ en tête du tube et le reste aux 2/3 de la longueur.

La proportion de télomères lourds ne dépasse pas 0,53 % avec un taux de conversion du $C_2F_4$ de 58,5 %.

**3c** : On répète l'essai 3b, mais en opérant à 355°C. Le taux de conversion du $C_2F_4$ remonte à 73,9 % pour un taux de télomères lourds égal à 0,76 %.

Le tableau suivant précise les conditions opératoires et les résultats de ces trois essais.

| ESSAI | 3a comparatif | 3b | 3c |
|---|---|---|---|
| % de $C_2F_4$ introduit en tête du réacteur | 100 | 50 | 50 |
| Rapport molaire global $(C_2F_5I + C_4F_9I)/C_2F_4$ | 1,27 | 1,31 | 1,22 |
| Temps de contact (secondes) | 59,5 | 59,9 | 60,2 |
| Température (°C) | 344 | 344 | 355 |
| Taux de conversion de $C_2F_4$ (%) | 76,6 | 58,5 | 73,9 |
| Répartition des télomères (% molaires) | | | |
| $C_4$ | 53,4 | 58,3 | 54,4 |
| $C_6$ | 25,0 | 25,9 | 27,2 |
| $C_8$ | 11,5 | 10,1 | 11,7 |
| $C_{10}$ | 6,0 | 4,05 | 4,46 |
| $C_{12}$ | 2,25 | 1,14 | 1,46 |
| > $C_{12}$ | 1,82 | 0,53 | 0,76 |

La comparaison des essais 3a et 3c montre qu'à taux de conversion sensiblement égal, l'introduction étagée du $C_2F_4$ permet de réduire avantageusement la proportion des télomères lourds difficilement valorisables.

**Revendications**

1. Procédé continu de préparation d'iodures de perfluoroalkyle contenant de 4 à 12 atomes de carbone par télomérisation thermique du tétrafluoroéthylène par l'iodure de pentafluoroéthyle ou d'heptafluoroisopropyle dans un réacteur tubulaire, caractérisé en ce qu'il comprend une double alimentation en tétrafluoroéthylène, la première en tête du réacteur et la seconde en au moins un point du réacteur situé entre les 2/5 et les 3/4 de la longueur du tube, la proportion de tétrafluoroéthylène alimentée en tête représentant 30 à 75 % de la quantité totale de tétrafluoroéthylène mise en oeuvre.

2. Procédé selon la revendication 1, dans lequel la seconde alimentation en tétrafluoroéthylène est effectuée en un point du réacteur situé environ aux 2/3 de la longueur du tube.

3. Procédé selon la revendication 1 ou 2, dans lequel la proportion de tétrafluoroéthylène alimentée en tête représente 40 à 60 % de la quantité totale de tétrafluoroéthylène mise en oeuvre.

4. Procédé selon l'une des revendications 1 à 3, dans lequel le rapport molaire global : télogène/$C_2F_4$ total est compris entre 1 et 3, de préférence entre 1,1 et 2.

5. Procédé selon l'une des revendications 1 à 4, dans lequel la télomérisation est effectuée à une température allant de 300 à 360°C, de préférence entre environ 325 et 355°C.

6. Procédé selon l'une des revendications 1 à 5, dans lequel on opère à la pression atmosphérique.

7. Procédé selon l'une des revendications 1 à 6, dans lequel le temps de contact est compris entre 10 et 70 secondes, de préférence entre 30 et 60 secondes.

8. Procédé selon l'une des revendications 1 à 7, dans lequel on recycle à l'entrée du réacteur l'iodure de pentatluoroéthyle ou d'heptafluoroisopropyle non transformé ou un mélange de celui-ci avec des télomères de rang moyen inférieur à celui des télomères désirés.

## Claims

1. Continuous process for the preparation of perfluoroalkyl iodides containing from 4 to 12 carbon atoms by thermal telomerisation of tetrafluoroethylene by pentafluoroethyl iodide or heptafluoroisopropyl iodide in a tubular reactor, characterised in that it comprises a twin feed of tetrafluoroethylene, the first feed at the top of the reactor and the second at at least one point in the reactor located between 2/5 and 3/4 of the length of the tube, the proportion of tetrafluoroethylene fed in at the top representing 30 to 75 % of the total amount of tetrafluoroethylene used.

2. Process according to Claim 1, in which the second feed of tetrafluoroethylene is effected at a point in the reactor located at approximately 2/3 of the length of the tube.

3. Process according to Claim 1 or 2, in which the proportion of tetrafluoroethylene fed in at the top represents 40 to 60 % of the total amount of tetrafluoroethylene used.

4. Process according to one of Claims 1 to 3, in which the overall molar ratio: telogen/total $C_2F_4$ is between 1 and 3, preferably between 1.1 and 2.

5. Process according to one of Claims 1 to 4, in which the telomerisation is carried out at a temperature ranging from 300 to 360 °C, preferably between about 325 and 355 °C.

6. Process according to one of Claims 1 to 5, in which the reaction is carried out under atmospheric pressure.

7. Process according to one of Claims 1 to 6, in which the contact time is between 10 and 70 seconds, preferably between 30 and 60 seconds.

8. Process according to one of Claims 1 to 7, in which the unconverted pentafluoroethyl iodide or heptafluoroisopropyl iodide or a mixture of the latter with telomers having an average class lower than that of the desired telomers is recycled to the reactor inlet.

## Patentansprüche

1. Kontinuierliches Verfahren zur Herstellungvon Perfluoralkyliodiden mit 4 bis 12 Kohlenstoffatomen durch thermische Telomerisation von Tetrafluorethylen mit Pentafluorethyliodid oder Heptafluorisopropyliodid in einem Röhrenreaktor, dadurch gekennzeichnet, daß es eine zweifache Zufuhr von Tetrafluorethylen umfaßt, wobei die erste Zufuhr am Kopf des Reaktors und die zweite an einer Stelle des Reaktors, welche zwischen 2/5 und 3/4 der Röhrenlänge gelegen ist, erfolgen und wobei die Menge des am Kopf zugesetzten Tetrafluorethylens 30 bis 75 % der Gesamtmenge des eingesetzten Tetrafluorethylens darstellt.

2. Verfahren nach Anspruch 1, in dem die zweite Zufuhr von Tetrafluorethylen an einer Stelle des Reaktors erfolgt, die bei etwa 2/3 der Röhrenlänge gelegen ist.

3. Verfahren nach Anspruch 1 oder 2, bei dem die Menge des am Kopf zugesetzten Tetrafluorethylens 40 bis 60 % der Gesamtmenge des eingesetzten Tetrafluorethylens darstellt.

4. Verfahren nach einem der Ansprüche 1 bis 3, bei dem das Gesamtmolverhältnis Telogen/Gesamt-$C_2F_4$ zwischen 1 und 3 liegt, vorzugsweise zwischen 1,1 und 2.

5. Verfahren nach einem der Ansprüche 1 bis 4, bei der die Telomerisation bei einer Temperatur von 300 bis 360 °C durchgeführt wird, vorzugsweise zwischen ca. 325 und 355 °C.

6. Verfahren nach einem der Ansprüche 1 bis 5, bei dem man bei Atmosphärendruck arbeitet.

7. Verfahren nach einem der Ansprüche 1 bis 6, bei dem die Kontaktzeit zwischen 10 und 70 Sekunden beträgt, vorzugsweise zwischen 30 und 60 Sekunden.

8. Verfahren nach einem der Ansprüche 1 bis 7, bei dem man bei Reaktoreintritt das nicht umgewandelte Pentafluorethyliodid oder Heptafluorisopropyliodid oder eine Mischung der Iodide mit Telomeren mittlerer Größe, welche kleiner sind als die gewünschten Telomere, dem Prozeß wieder zuführt.